Europäisches Patentamt

European Patent Office

Office européen des brevets

(19)

(11) Veröffentlichungsnummer : **0 081 856**
**B1**

(12) **EUROPÄISCHE PATENTSCHRIFT**

(45) Veröffentlichungstag der Patentschrift :
**13.03.85**

(21) Anmeldenummer : **82111648.0**

(22) Anmeldetag : **15.12.82**

(51) Int. Cl.⁴ : **C 07 C 43/313**, C 07 C 41/54,
C 07 C 41/58

(54) **Verfahren zur Herstellung von Chloracetaldehyddimethylacetal.**

(30) Priorität : **15.12.81 DE 3149652**

(43) Veröffentlichungstag der Anmeldung :
**22.06.83 Patentblatt 83/25**

(45) Bekanntmachung des Hinweises auf die Patenterteilung : **13.03.85 Patentblatt 85/11**

(84) Benannte Vertragsstaaten :
**CH DE FR GB LI**

(56) Entgegenhaltungen :
**GB-A- 1 158 982**
**US-A- 2 330 570**

(73) Patentinhaber : **WACKER-CHEMIE GMBH**
**Prinzregentenstrasse 22**
**D-8000 München 22 (DE)**

(72) Erfinder : **Deinhammer, Wolfgang, Dr. Dipl.-Ing.**
**Röntgenstrasse 32**
**D-8263 Burghausen (DE)**

**Beschreibung**

Gegenstand der Erfindung ist ein verbessertes Verfahren zur Herstellung von Chloracetaldehyddimethylacetal durch Umsetzung von Vinylacetat und Chlor in methanolischer Lösung.

Es ist bekannt, Chloracetaldehydacetale durch Chlorieren von Vinylverbindungen in alkoholischen Medium herzustellen (vgl. Ullmann Enzyklopädie der technischen Chemie, 4. Auflage 1975, Bd. 9, S. 375). Bei Einsatz von Vinylchlorid entstehen jedoch beträchliche Mengen an 1,1,2-Trichlorethan als Nebenprodukt (vgl. US-PS 2 803 668), das aufgrund seines ähnlichen Siedepunkts nur schwierig von dem Chloracetaldehyddimethylacetal abzutrennen ist. Dieses Verfahren kann auch unter Zusatz von säurebindenden Reagenzien vom Typus der Hydroxide, Oxide, Carbonate und Alkoholate von Metallen der Gruppe Ia und IIa des Periodensystems durchgeführt werden, wodurch das Reaktionsgleichgewicht zu Gunsten der gewünschten Acetalverbindung verschoben und das 1,1,2-Trichlorethan in 1,1-Dichlorethylen übergeführt werden sollen (vgl. DE-OSS 16 43 899 und 16 93 017, die der US-PS 3 784 612 entspricht). Die Durchführung derartiger Verfahren im großtechnischen Bereich ist indessen stets mit aufwendigen Sicherheitsvorkehrungen für das hochtoxische Vinylchlorid belastet.

Bei Einsatz von Vinylether ist die Gegenwart von alkalischen Mitteln, wie Oxiden, Hydroxiden, Alkoholaten, Carbonaten und Bicarbonaten von Alkali- oder Erdalkalimetallen schon während der Chlorierung zur Erzielung der angegebenen Ausbeuten erforderlich (vgl. US-PS 2 550 637), wobei sich Calciumoxid aufgrund seiner begrenzten Löslichkeit in Methanol besonders bewährt hat (vgl. US-PS Nr. 3 379 772). Die Durchführung dieser Verfahren im großtechnischen Maßstab ist jedoch unwirtschaftlich, da die Ausgangsverbindungen schwer zugänglich, für die Verfahrensführung Temperaturen unterhalb von 0 °C erforderlich und die Ausbeuten an dem gewünschten Acetal gering sind.

Die Umsetzung von Vinylacetat und Halogen in Gegenwart von überschüssigem Alkohol ist ebenfalls seit langem bekannt (vgl. US-PS 2 330 570 und 2 411 826, sowie Am. Chem. Soc., Vol. 61, (1939), S. 1705-1706 von E. M. Filachione). Hierbei wurden die Acetale nach üblichen Methoden aus dem Reaktionsgemisch isoliert, beispielsweise durch Zugabe von Wasser und anschließender Extraktion der Acetalschicht mit einem mit Wasser nichtmischbaren Lösungsmittel, wie Ether, Chloroform oder Benzol. Der organische Extrakt wurde zur Entfernung von sauren oder anderen wasserlöslichen Nebenprodukten mit Wasser und/oder einer wäßrigen alkalischen Lösung, wie einer Natriumbicarbonatlösung gewaschen, dann wurde das organische Lösungsmittel abdestilliert und das Acetal durch Destillation gereinigt. Es wurde jedoch bereits von E. M. Filachione festgestellt, daß die Ausbeuten von so gewonnenen Methylhaloacetalen nur bei etwa 46-53 % liegen und somit beträchtlich niedriger sind als diejenigen der entsprechenden Ethylhaloacetale. Für die Durchführung dieses Verfahrens zur Herstellung von Chloracetaldehyddimethylacetal in großtechnischem Maßstab auf Basis des leicht verfügbaren und nicht toxischen Vinylacetats ist jedoch diese umständliche Aufarbeitungsmethode nicht nur wegen der erzielten schlechten Ausbeute unwirtschaftlich, sondern auch verfahrenstechnisch, weil große Lösungsmittelmengen destilliert werden müssen und die Rückgewinnung des Methanols aus der wäßrigen Phase zusätzlichen Aufwand erfordert.

Es stellt sich somit die Aufgabe, ein verbessertes Verfahren zur Herstellung von Chloracetaldehyddimethylacetal durch Umsetzung von Vinylacetat und Chlor in stöchiometrischen Mengen in Gegenwart von überschüssigem Methanol bei Temperaturen < 20 °C zur Verfügung zu stellen, das die Gewinnung der gewünschten Verbindung in hohen Ausbeuten und die Aufarbeitung des Reaktionsgemisches ohne die Zugabe von Wasser und von mit Wasser nicht mischbaren organischen Lösungsmitteln ermöglicht.

Diese Aufgabe wird erfindungsgemäß dadurch gelöst, daß aus dem Reaktionsgemisch nach beendeter Chlorzugabe niedrig siedende Anteile mit Siedepunkten bis zu 60 °C unter dem Druck der umgebenden Atmosphäre, worunter ein Druck von etwa 0,1 MPa zu verstehen ist, ganz oder teilweise abdestilliert werden, der flüssige Rückstand durch Zugabe von Feststoffen aus der Gruppe der Oxide und Carbonate von Metallen der Gruppe IIa des Periodensystems unter Einhaltung einer Temperatur von 20 °-60 °C neutralisiert, von den so gebildeten beiden Phasen die organische odere Schicht abgetrennt und das erhaltene Rohprodukt durch Destillation gereinigt wird.

Für die Durchführung des erfindungsgemäßen Verfahrens werden stöchiometrische Mengen von Vinylacetat und Chlor vorteilhaft gleichzeitig in vorgelegtes Methanol eingetragen, wobei etwa 4-6 Mol Methanol je Mol Vinylacetat verwendet werden und durch ausreichende Kühlung, kräftige Bewegung und Regelung der Zugabegeschwindigkeit dafür gesorgt wird, daß die Reaktionstemperatur 20 °C nicht übersteigt. Reaktionstemperaturen im Bereich von 0 °-10 °C sind dabei bevorzugt. Nach beendeter Chlorzugabe erfolgt die Aufarbeitung des Reaktionsgemisches unter den definierten Bedingungen, die im einzelnen wie folgt näher erläutert werden :

Durch das Abdestillieren der niedrig siedenden Anteile wird der größte Teil des als Nebenprodukt gebildeten Chlorwasserstoffes entfernt und somit der störenden Einwirkung auf das im sauren Bereich empfindliche Chloracetaldehyddimethylacetal entzogen. Gleichzeitig werden von dem als Nebenprodukt entstandenen Methylacetat etwa 2/3, d. h. etwa 50-80 Gew.% entfernt, das mit Methanol im Gewichtsverhältnis 80/20 ein bei 55 °C siedendes azeotropes Gemisch bildet, wodurch ebenfalls das Reaktionsgleichgewicht zugunsten der gewünschten Verbindung verschoben wird.

Der nach dem Abdestillieren der niedrig siedenden Anteile verbleibende flüssige Rückstand wird unmittelbar anschliessend mit den Feststoffen der definierten Art versetzt, wobei sich Oxide und Carbonate von Calcium und Magnesium aufgrund ihrer leichten Verfügbarkeit besonders bewährt haben. Die Feststoffe können in Form von Pulvern oder Granulaten verwendet werden, deren Teilchengröße nicht entscheidend ist. Zur Erzielung einer raschen Reaktion ist es indessen vorteilhaft, wenn die Feststoffe Teilchengrößen von $\leqslant$ 0,5 mm haben und wenn durch kräftige Bewegung, beispielsweise durch Rühren für deren Verteilung in dem flüssigen Rückstand gesorgt wird. Die Feststoffmengen sind hierbei so bemessen, daß diese für die Neutralisation des flüssigen Rückstandes ausreichen, d. h. daß ein wäßriger Extrakt desselben einen pH-Wert von > 5 aufweist. Die Zugabegeschwindigkeit der Feststoffe wird in Abhängigkeit von der zur Verfügung stehenden Kühlfläche so geregelt, daß der angegebene Temperaturbereich eingehalten wird, der vorzugsweise im Bereich von 30° bis 50 °C liegt.

Nach beendeter Neutralisation liegt das Reaktionsgemisch in Form von 2 getrennten flüssigen Phasen vor, wobei die untere Schicht hauptsächlich das während der Neutralisation gebildete Calciumchlorid, gelöst in Methanol, und die obere, organische Schicht die Hauptmenge des rohen Chloracetaldehyddimethylacetals enthält. Nach Trennung der beiden Phasen in üblicher Weise kann gegebenenfalls die untere Schicht mit Methylacetat extrahiert werden.

Die obere Schicht wird dann, gegebenenfalls zusammen mit dem Methylacetatextrakt aus der unteren Schicht fraktioniert destilliert, wobei als Hauptfraktion reines Chloracetaldehyddimethylacetal in Ausbeuten von > 90 %, bezogen auf eingesetztes Vinylacetat gewonnen wird, das ein wertvolles Zwischenprodukt für die Herstellung von Riechstoffen, Arzneimitteln und Pflanzenschutzmitteln ist. Durch das folgende Beispiel wird das erfindungsgemäße Verfahren näher erläutert.

Beispiel

In 324 ml Methanol wurden unter Rühren und Kühlen gleichzeitig 186 ml (172 g = 2,0 Mol) Vinylacetat und 142 g (2,0 Mol) Chlor bei 0° bis 2° innerhalb von 1 Std. eingetragen. Dann wurden über eine 50 cm Vigreux-Kolonne 50 ml Leichtsieder abdestilliert, anschließend 19,8 g eines Zwischenlaufs eines gleich durchgeführten Ansatzes zugegeben, schließlich unter Rühren und Kühlen bei 40°-50° 34 g Calciumoxid innerhalb von 30 Min. eingetragen. Nach beendeter Neutralisation lag das Reaktionsgemisch in Form von zwei getrennten, flüssigen Phasen vor. Nach Abtrennung der Schichten wurde die erhaltene obere Schicht auffraktioniert, wobei 88 g Vorlauf, der hauptsächlich Methylacetat und Methanol, 19,8 g Zwischenlauf, der hauptsächlich Methanol, Chloracetaldehyd, Chloracetaldehydmono- sowie -dimethylacetal enthielt und als Hauptfraktion 223,1 g Chloracetaldehyddimethylacetal d. s. 89,5 % d. Th. erhalten wurden. Wird die nach der Calciumoxidzugabe entstehende untere Schicht noch 2x mit je 100 ml Methylacetat ausgeschüttelt und die vereinigten organischen Phasen destilliert, werden 92,1 % d. Th. Chloracetaldehyddimethylacetal erhalten.

## Anspruch

Verfahren zur Herstellung von Chloracetaldehyddimethylacetal durch Umsetzung von Vinylacetat und Chlor in stöchiometrischen Mengen in Gegenwart von überschüssigem Methanol bei Temperaturen < 20 °C und anschließender Abtrennung des Chloracetaldehyddimethylacetals aus dem Reaktionsgemisch, dadurch gekennzeichnet, daß aus dem Reaktionsgemisch nach beendeter Chlorzugabe niedrig siedende Anteile mit Siedepunkten bis zu 60 °C unter dem Druck der umgebenden Atmosphäre ganz oder teilweise abdestilliert werden, der flüssige Rückstand durch Zugabe von Feststoffen aus der Gruppe der Oxide und Carbonate von Metallen der Gruppe IIa des Periodensystems unter Einhaltung einer Temperatur von 20° bis 60 °C neutralisiert, von den so gebildeten beiden Phasen die organische obere Schicht abgetrennt und das erhaltene Rohrprodukt durch Destillation gereinigt wird.

## Claim

Process for the manufacture of chloroacetaldehyde dimethyl acetal by reacting vinyl acetate and chlorine in stoichiometric amounts in the presence of excess methanol at temperatures of less than 20 °C, and subsequently separating off the chloroacetaldehyde dimethyl acetal from the reaction mixture, characterised in that low-boiling constituents having boiling points of up to 60 °C are distilled off, completely or partially, from the reaction mixture when the addition of chlorine is complete, under the pressure of the surrounding atmosphere, the liquid residue is neutralised by the addition of solid substances selected from the group consisting of the oxides and carbonates of metals of Group IIa of the Periodic Table while maintaining a temperature of from 20 to 60 °C, the organic upper layer is separated off from the two phases so formed, and the resulting crude product is purified by distillation.

## Revendication

Procédé de préparation du diméthyl-acétal du choracétaldéhyde par réaction de l'acétate de vinyle et du chlore en des quantités stœchiométriques en présence d'un excès de méthanol à des températures inférieures à 20 °C, puis séparation du diméthyl-acétal du chloracétaldéhyde à partir du mélange réactionnel, procédé caractérisé en ce que, l'addition du chlore terminée, on élimine du mélange réactionnel, totalement ou partiellement, les constituants dont les points d'ébullition ne dépassent pas 60 °C, par distillation sous la pression de l'atmosphère environnante, on neutralise le résidu liquide par addition de corps solides pris dans l'ensemble constitué par les oxydes et les carbonates des métaux du groupe IIa de la classification périodique, cela tout en maintenant une température de 20 à 60 °C, on sépare la couche organique supérieure du système à deux phases ainsi formé et on purifie par distillation le produit brut obtenu.